# EUROPÄISCHE PATENTSCHRIFT

(11) **EP 4 633 549 B1**
(45) Veröffentlichungstag und Bekanntmachung des Hinweises auf die Patenterteilung: **29.07.2026**
(21) Anmeldenummer: 23809570.7
(22) Anmeldetag: 20.11.2023
(51) Int. Cl.: A61F 5/01, A61F 5/02

(54) **KREUZUNGSELEMENT FÜR ORTHOPÄDISCHE UND SPORT-HILFSMITTEL**
CROSSING ELEMENT FOR ORTHOPEDIC AND SPORTS AID DEVICES
ÉLÉMENT DE CROISEMENT POUR DISPOSITIFS ORTHOPÉDIQUES ET D'AIDE AU SPORT

(30) Priorität: 13.12.2022 DE 102022213572
(43) Veröffentlichungstag der Anmeldung: 22.10.2025
(73) Patentinhaber: Bauerfeind AG, 07937 Zeulenroda-Triebes (DE)
(72) Erfinder: STIER, Gerald, 07937 Zeulenroda-Triebes (DE); HEBENSTREIT, Sandro, 07937 Zeulenroda-Triebes (DE); BAUERFEIND-JOHNSON, Beatrix, 07937 Zeulenroda-Triebes (DE)
(74) Vertreter: Gleiss Große Schrell und Partner mbB
(86) Internationale Anmeldenummer: PCT/EP2023/082436
(87) Internationale Veröffentlichungsnummer: WO 2024/125967

(56) Entgegenhaltungen:
- US-A1- 2019 053 930
- US-A1- 2021 369 481

## Beschreibung

Die vorliegende Erfindung betrifft das technische Gebiet der orthopädischen und Sport-Hilfsmittel. Die Erfindung stellt insbesondere ein Mittel zur kontrollierten und insbesondere reibungsarmen Kreuzung mindestens eines Spannungselements in an den menschlichen oder tierischen Körper angelegten orthopädischen und Sport-Hilfsmitteln bereit.

Ein besonderes Problem bei der Führung von Spannungselementen in orthopädischen und Sport-Hilfsmitteln besteht an den Stellen, an denen sich unterschiedliche Spannungselemente oder Abschnitte eines Spannungselements kreuzen. Ohne eine Kontrolle des Verlaufs des mindestens einen Spannungselements an den Kreuzungspunkten kommt es zu einer Berührung der unterschiedlichen Spannungselemente oder der Abschnitte des zusammenhängenden Spannungselements und damit verbunden zu Reibungskräften, die zu einer deutlichen Beeinträchtigung der Kraftübertragung und zu einem unerwünschten Verschleiß der Spannungselemente führen. So betrifft die US 2021/0369481 A1 aus dem Stand der Technik beispielsweise ein Kabelsystem für Knieorthesen, das in einer Weise entlang der Orthese geführt wird, die es ermöglicht, das vordere Kreuzband (ACL) und das Innenband (MCL) des Knies zu unterstützen und durch exzessive Bewegungen des Kniegelenks hervorgerufene Verletzungen der Bänder zu vermeiden. In gewissen Ausführungsformen wird zu diesem Zweck eine Kabelführung verwendet, bei der sich zwei Kabel oder zwei Abschnitte eines Kabels in einem Punkt kreuzen. Bei einer ungeführten Kreuzung unterschiedlicher Spannungselemente oder von Abschnitten eines Spannungselements können von außen auf den Kreuzungspunkt wirkende Kräfte zu einer Blockierung des mindestens einen Spannungselements führen. Damit verbunden kann es gegebenenfalls auch zu einer Beeinträchtigung der Beweglichkeit von orthopädischen und Sport-Hilfsmitteln kommen. Verhindert wird dies im Stand der Technik durch die Führung von Spannungselementen unter Vermeidung von Kreuzungspunkten, insbesondere durch die Verwendung von Umlenkschnallen, -ringen, oder -laschen. Ziel der vorliegenden Erfindung ist es daher Mittel zur kontrollierten und insbesondere reibungsarmen Kreuzung mindestens eines Spannungselements in orthopädischen und Sport-Hilfsmitteln bereitzustellen.

Die vorliegende Erfindung löst das ihr zugrundliegende technische Problem durch den Gegenstand der unabhängigen Ansprüche.

Die vorliegende Erfindung betrifft insbesondere ein Kreuzungselement für orthopädische oder Sport-Hilfsmittel, umfassend mindestens einen ersten Führungsbereich zur Aufnahme eines Spannungselements des Hilfsmittels und mindestens einen zweiten Führungsbereich zur Aufnahme eines Spannungselements des Hilfsmittels, wobei das Kreuzungselement dadurch gekennzeichnet ist, dass die mindestens zwei Führungsbereiche des Kreuzungselements so ausgestaltet und zueinander angeordnet sind, dass sich ein in dem ersten Führungsbereich geführtes Spannungselement und ein in dem zweiten Führungsbereich geführtes Spannungselement in mindestens einem Punkt berührungsfrei kreuzen. Insbesondere verläuft das von den Führungsbereichen des Kreuzungselements geführte mindestens eine Spannungselement zumindest in dem mindestens einen Kreuzungspunkt in unterschiedlichen Ebenen.

In besonders bevorzugter Ausführungsform der vorliegenden Erfindung handelt es sich bei dem Kreuzungselement für orthopädische und Sport-Hilfsmittel um ein Kreuzungselement für Orthesen, insbesondere für eine Rückenorthese, Hüftorthese, Kniegelenkorthese, Ellenbogenorthese, Armorthese oder Sprunggelenkorthese.

Gemäß einer bevorzugter Ausführungsform der vorliegenden Erfindung handelt es sich bei dem Kreuzungselement für orthopädische und Sport-Hilfsmittel um ein Kreuzungselement für Bandagen, insbesondere für eine Rückenbandage, Hüftbandage, Kniegelenkbandage, Ellenbogenbandage, Armbandage oder Sprunggelenkbandage.

In einer weiteren bevorzugten Ausführungsform der vorliegenden Erfindung handelt es sich bei dem Kreuzungselement für orthopädische und Sport-Hilfsmittel um ein Kreuzungselement für Sport-Hilfsmittel, insbesondere für Handschuhe und Schuhe, bevorzugte für Sportschuhe, insbesondere für Joggingschuhe, Wanderschuhe, Fahrradschuhe, Skischuhe oder Snowboard Boots.

In einer bevorzugten Ausführungsform ist das Kreuzungselement aus mindestens einem Kunststoff oder einem Metall, insbesondere einem Leichtmetall, gefertigt. Bevorzugt ist der mindestens eine Kunststoff ausgewählt aus der Gruppe bestehend aus Polystyrol (PS), Polycarbonat (PC), Polypropylen (PP), Polyethylenterephthalat (PET), Cycloolefine (COC), Polyamid (PA), Polyketon (PK), Acrylnitril-Butadien-Styrol (ABS), Polyethylen (PE), thermoplastisches Polyurethan (TPU), thermoplastische Elastomere (TPE) und Mischungen davon.

In einer bevorzugten Ausführungsform ist das Kreuzungselement aus mindestens zwei, bevorzugt aus mindestens drei, bevorzugt aus mindestens vier, unterschiedlichen Materialien, insbesondere aus mindestens zwei, bevorzugt aus mindestens drei, bevorzugt aus mindestens vier, unterschiedlichen Kunststoffen, gefertigt.

Erfindungsgemäß umfasst das Kreuzungselement mindestens zwei Führungsbereiche. In einer bevorzugten Ausführungsform der vorliegenden Erfindung kann vorgesehen sein, dass das Kreuzungselement mindestens drei, bevorzugt mindestens vier, bevorzugt mindestens fünf, Führungsbereiche aufweist. Bevorzugt umfasst das erfindungsgemäße Kreuzungselement höchstens fünf, bevorzugt höchstens vier, bevorzugt höchstens drei, Führungsbereiche. In einer besonders bevorzugten Ausführungsform umfasst das Kreuzungselement zwei, bevorzugt drei, bevorzugt vier, bevorzugt fünf, Führungsbereiche.

Entsprechend kreuzen sich in dem erfindungsgemäßen Kreuzungselement mindestens zwei Spannungselemente, insbesondere zwei Spannungselemente, eines orthopädischen oder Sport-Hilfsmittels berührungsfrei. Erfindungsgemäß kann vorgesehen sein, dass sich in dem Kreuzungselement mindestens drei, mindestens vier oder mindestens fünf Spannungselemente, insbesondere drei, vier oder fünf Spannungselemente, eines orthopädischen oder Sport-Hilfsmittels berührungsfrei kreuzen.

Gemäß einer bevorzugten Ausführungsform der vorliegenden Erfindung handelt es sich bei mindestens einem Führungsbereich des Kreuzungselements, insbesondere bei dem ersten und/oder dem zweiten Führungsbereich des Kreuzungselements, um einen Führungskanal. Dabei kann der Führungskanal offen oder geschlossen ausgestaltet sein.

Bevorzugt ist mindestens ein Führungsbereich, insbesondere der erste und/oder der zweite Führungsbereich, insbesondere der erste und/oder der zweite Führungskanal, des Kreuzungselements als ein offener Führungskanal, insbesondere als eine Nut, ausgebildet. Besonders bevorzugt ist mindestens ein Führungsbereich, insbesondere der erste und/oder der zweite Führungsbereich, insbesondere der erste und/oder der zweite Führungskanal, des Kreuzungselements als eine sich zu dem mindestens einen Kreuzungspunkt hin verjüngende Nut ausgebildet.

In einer bevorzugten Ausführungsform der vorliegenden Erfindung ist mindestens ein Führungsbereich, insbesondere der erste und/oder zweite Führungsbereich, so ausgeformt, dass ein in dem mindestens einen Führungsbereich, insbesondere in dem ersten und/oder zweiten Führungsbereich, geführtes Spannungselement zumindest teilweise umgriffen wird. In einer weiteren Ausführungsform kann mindestens ein Führungsbereich, insbesondere der erste und/oder zweite Führungsbereich, so ausgeformt sein, dass ein in dem mindestens einen Führungsbereich, insbesondere in dem ersten und/oder zweiten Führungsbereich, geführtes Spannungselement vollständig umgriffen wird. Dabei kann erfindungsgemäß vorgesehen sein, dass ein in mindestens einen Führungsbereich, insbesondere in dem ersten und/oder zweiten Führungsbereich, des Kreuzungselements geführtes Spannungselement über die gesamte Länge des mindestens einen Führungsbereichs, insbesondere des ersten und/oder zweiten Führungsbereichs, oder abschnittsweise umgriffen wird, insbesondere teilweise oder vollständig umgriffen wird.

Erfindungsgemäß kann auch vorgesehen sein, dass es sich bei mindestens einem Führungsbereich, insbesondere bei dem ersten und/oder dem zweiten Führungsbereich, insbesondere dem ersten und/oder dem zweiten Führungskanal, des Kreuzungselements um einen geschlossenen Kanal handelt.

Besonders bevorzugt ist der erste Führungsbereich so ausgestaltet, dass ein in dem ersten Führungsbereich geführtes Spannungselement des Hilfsmittels in dem mindestens einen Kreuzungspunkt über einem in dem zweiten Führungsbereich geführten Spannungselement des Hilfsmittels verläuft.

In einer weiteren bevorzugten Ausführungsform ist der erste Führungsbereich so ausgestaltet, dass ein in dem ersten Führungsbereich geführtes Spannungselement des Hilfsmittels in dem mindestens einen Kreuzungspunkt unter einem in dem zweiten Führungsbereich geführten Spannungselement des Hilfsmittels verläuft.

In einer besonders bevorzugten Ausführungsform der vorliegenden Erfindung liegen die Führungsbereiche des Kreuzungselements, insbesondere der erste und der zweite Führungsbereich, in unterschiedlichen Ebenen.

Bevorzugt liegen die Führungsbereiche des Kreuzungselements, insbesondere der erste und der zweite Führungsbereich des Kreuzungselements, zumindest im Bereich mindestens eines Kreuzungspunktes eines in dem ersten Führungsbereich geführten Spannungselements des Hilfsmittels mit einem in dem zweiten Führungsbereich geführten Spannungselement des Hilfsmittels in unterschiedlichen Ebenen.

Besonders bevorzugt liegen die Führungsbereiche des Kreuzungselements, insbesondere der erste und zweite Führungsbereich des Kreuzungselements, in allen Kreuzungspunkten der Spannungselemente des Hilfsmittels, insbesondere in allen Kreuzungspunkten eines in dem ersten Führungsbereich geführten Spannungselements des Hilfsmittels mit einem in dem zweiten Führungsbereich geführten Spannungselement des Hilfsmittels, in unterschiedlichen Ebenen, insbesondere in mindestens zwei unterschiedlichen Ebenen.

In einer bevorzugten Ausführungsform der vorliegenden Erfindung sind die Führungsbereiche des Kreuzungselements in unterschiedlichen Ebenen angeordnet. Besonders bevorzugt sind die Führungsbereiche des Hilfsmittels insbesondere im Bereich von mindestens einem Kreuzungspunkt, bevorzugt im Bereich von mindestens zwei Kreuzungspunkten, bevorzugt im Bereich von mindestens drei Kreuzungspunkten, insbesondere im Bereich aller Kreuzungspunkte, der Spannungselemente des Hilfsmittels, in unterschiedlichen Ebenen angeordnet. Bevorzugt sind die Führungsbereiche des Kreuzungselements in mindestens zwei, bevorzugt mindestens drei, bevorzugt mindestens vier, bevorzugt mindestens fünf, unterschiedlichen Ebenen angeordnet.

Gemäß einer bevorzugten Ausführungsform der vorliegenden Erfindung ist der erste Führungsbereich über dem zweiten Führungsbereich des Kreuzungselements angeordnet. Bevorzugt ist der erste Führungsbereich zumindest im Bereich mindestens eines Kreuzungspunktes eines in dem ersten Führungsbereich geführten Spannungselements des Hilfsmittels mit einem in dem zweiten Führungsbereich geführten Spannungselement des Hilfsmittels über dem zweiten Führungsbereich angeordnet. Gemäß dieser bevorzugten Ausführungsform der Erfindung wird an dem mindestens einen Kreuzungspunkt ein in dem ersten Führungsbereich des Kreuzungselements geführtes Spannungselement berührungsfrei über ein in dem zweiten Führungsbereich des Kreuzungselements geführtes Spannungselement geführt.

Besonders bevorzugt liegt der erste Führungsbereich zumindest im Bereich mindestens eines Kreuzungspunktes eines in dem ersten Führungsbereich geführten Spannungselements des Hilfsmittels mit einem in dem zweiten Führungsbereich geführten Spannungselement des Hilfsmittels mindestens 0,25 cm, bevorzugt mindestens 0,5 cm, bevorzugt mindestens 0,75 cm, bevorzugt mindestens 1 cm, bevorzugt mindestens 1,25 cm, bevorzugt mindestens 1,5 cm, bevorzugt mindestens 1,75 cm, bevorzugt mindestens 2 cm, bevorzugt mindestens 2,25 cm, bevorzugt mindestens 2,5 cm, über dem zweiten Führungsbereich.

In einer weiteren bevorzugten Ausführungsform der Erfindung liegt der erste Führungsbereich zumindest im Bereich mindestens eines Kreuzungspunktes eines in dem ersten Führungsbereich geführten Spannungselements des Hilfsmittels mit einem in dem zweiten Führungsbereich geführten Spannungselement des Hilfsmittels höchstens 3 cm, bevorzugt höchstens 2,75 cm, bevorzugt höchstens 2,5 cm, bevorzugt höchstens 2 cm, bevorzugt höchstens 1,75 cm, bevorzugt höchstens 1,5 cm, bevorzugt höchstens 1,25 cm, bevorzugt höchstens 1 cm, bevorzugt höchstens 0,75 cm, bevorzugt höchstens 0,5 cm, über dem zweiten Führungsbereich.

Gemäß einer bevorzugten Ausführungsform liegt der erste Führungsbereich zumindest im Bereich mindestens eines Kreuzungspunktes eines in dem ersten Führungsbereich geführten Spannungselements des Hilfsmittels mit einem in dem zweiten Führungsbereich geführten Spannungselement des Hilfsmittels 0,25 cm bis 3 cm, bevorzugt 0,25 cm bis 2,5 cm, bevorzugt 0,5 cm bis 2 cm, bevorzugt 0,5 cm bis 1,5 cm, bevorzugt 0,75 cm bis 1 cm, über dem zweiten Führungsbereich.

Bevorzugt umfasst mindestens ein Führungsbereich, insbesondere der erste oder zweite Führungsbereich, mindestens eine Rampe, bevorzugt zwei Rampen. In einer bevorzugten Ausführungsform der vorliegenden Erfindung wird das im ersten Führungsbereich verlaufende Spannungselement des Hilfsmittels mittels mindestens einer Rampe, bevorzugt mittels zweier Rampen, über das im zweiten Führungsbereich verlaufende Spannungselement des Hilfsmittels geführt.

In einer besonders bevorzugten Ausführungsform der vorliegenden Erfindung umfasst der erste Führungsbereich zwei Rampen, wobei der zweite Führungsbereich zwischen den zwei Rampen des ersten Führungsbereichs verläuft, sodass ein im ersten Führungsbereich verlaufendes Spannungselement mittels der zwei Rampen über ein im zweiten Führungsbereich verlaufendes Spannungselement geführt wird.

In einer weiteren Ausführungsform der vorliegenden Erfindung umfasst mindestens ein Führungsbereich, insbesondere der erste oder zweite Führungsbereich, mindestens eine Brücke. Bevorzugt wird das im ersten Führungsbereich verlaufende Spannungselement des Hilfsmittels mittels der Brücke über das im zweiten Führungsbereich verlaufende Spannungselement des Hilfsmittels geführt.

In einer bevorzugten Ausführungsform der vorliegenden Erfindung verlaufen die Führungsbereiche des Kreuzungselements, insbesondere der erste und zweite Führungsbereich, orthogonal zueinander. Besonders bevorzugt kreuzen sich das in dem ersten Führungsbereich geführte Spannungselement des Hilfsmittels und das in dem zweiten Führungsbereich geführte Spannungselement des Hilfsmittels in mindestens einem Punkt orthogonal. Erfindungsgemäß kreuzen sich die in den Führungsbereichen des Kreuzungselements geführten Spannungselemente des Hilfsmittels, insbesondere das in dem ersten Führungsbereich geführte Spannungselement des Hilfsmittels und das in dem zweiten Führungsbereich geführte Spannungselement des Hilfsmittels, berührungsfrei.

Besonders bevorzugt sind der erste und der zweite Führungsbereich des Kreuzungselements so zueinander angeordnet, dass sich ein in dem ersten Führungsbereich geführtes Spannungselement des Hilfsmittels und ein in dem zweiten Führungsbereich geführtes Spannungselement des Hilfsmittels in dem Kreuzungselement mit einem Schnittwinkel α von mindestens 20°, bevorzugt mindesten 25°, bevorzugt mindestens 30°, bevorzugt mindestens 35°, bevorzugt mindestens 40°, bevorzugt mindestens 45°, bevorzugt mindestens 50°, bevorzugt mindestens 55°, bevorzugt mindestens 60°, bevorzugt mindestens 65°, bevorzugt mindestens 70°, bevorzugt mindestens 75°, bevorzugt mindestens 80°, bevorzugt mindestens 85°, bevorzugt 90°, kreuzen.

Gemäß einer bevorzugten Ausführungsform der vorliegenden Erfindung sind der erste und der zweite Führungsbereich des Kreuzungselements so zueinander angeordnet, dass sich ein in dem ersten Führungsbereich geführtes Spannungselement des Hilfsmittels und ein in dem zweiten Führungsbereich geführtes Spannungselement des Hilfsmittels in dem Kreuzungselement mit einem Schnittwinkel α von höchstens 90°, bevorzugt höchstens 85°, bevorzugt höchstens 80°, bevorzugt höchstens 75°, bevorzugt höchstens 70°, bevorzugt höchstens 65°, bevorzugt höchstens 60°, bevorzugt höchstens 55°, bevorzugt höchstens 45°, bevorzugt höchstens 40°, bevorzugt höchstens 35°, bevorzugt höchstens 30°, bevorzugt höchstens 25°, kreuzen.

In einer weiteren bevorzugten Ausführungsform der vorliegenden Erfindung sind der erste und der zweite Führungsbereich des Kreuzungselements so zueinander angeordnet, dass sich ein in dem ersten Führungsbereich geführtes Spannungselement des Hilfsmittels und ein in dem zweiten Führungsbereich geführtes Spannungselement des Hilfsmittels in dem Kreuzungselement mit einen Schnittwinkel α von 20° bis 90°, bevorzugt 25° bis 90°, bevorzugt 30° bis 90°, bevorzugt 35° bis 90°, bevorzugt 40° bis 90°, bevorzugt 45° bis 90°, bevorzugt 50° bis 90°, bevorzugt 55° bis 90°, bevorzugt 60° bis 90°, bevorzugt 65° bis 90°, bevorzugt 70° bis 90°, bevorzugt 75° bis 90°, bevorzugt 80° bis 90°, bevorzugt 85° bis 90°, kreuzen.

In einer weiteren bevorzugten Ausführungsform der vorliegenden Erfindung sind der erste und der zweite Führungsbereich des Kreuzungselements so zueinander angeordnet, dass sich ein in dem ersten Führungsbereich geführtes Spannungselement des Hilfsmittels und ein in dem zweiten Führungsbereich geführtes Spannungselement des Hilfsmittels in dem Kreuzungselement mit einem Schnittwinkel α von 20° bis 90°, bevorzugt 25° bis 85°, bevorzugt 30° bis 80°, bevorzugt 35° bis 75°, bevorzugt 40° bis 70°, bevorzugt 45° bis 65°, bevorzugt 50° bis 60°, kreuzen.

In einer bevorzugten Ausführungsform der vorliegenden Erfindung sind die mindestens zwei Führungsbereiche des Kreuzungselements so ausgestaltet und zueinander angeordnet, dass sich die in den Führungsbereichen verlaufende Spannungselemente des Hilfsmittels in mindestens zwei, bevorzugten in mindestens drei, bevorzugt in mindestens vier, Punkten berührungsfrei kreuzen.

Gemäß einer weiteren bevorzugten Ausführungsform der vorliegenden Erfindung sind die mindestens zwei Führungsbereiche des Kreuzungselements so ausgestaltet und zueinander angeordnet, dass sich die in den Führungsbereichen geführten Spannungselemente des Hilfsmittels in höchstens vier, bevorzugt in höchstens drei, bevorzugt in höchstens zwei, Punkten berührungsfrei kreuzen.

Besonders bevorzugt sind die mindestens zwei Führungsbereiche des Kreuzungselements so ausgestaltet und zueinander angeordnet, dass sich die in den Führungsbereichen geführten Spannungselemente des Hilfsmittels in einem Punkt, bevorzugt in zwei Punkten, bevorzugten in drei Punkten, bevorzugt in vier Punkten, berührungsfrei kreuzen.

Gemäß einer bevorzugten Ausführungsform der vorliegenden Erfindung weist das Kreuzungselement mindestens eine Abdeckung auf. Besonders bevorzugt weist das Kreuzungselement zumindest über dem mindestens einen Kreuzungspunkt eines in dem ersten Führungsbereich geführten Spannungselements mit einem in dem zweiten Führungsbereich geführten Spannungselement mindestens eine Abdeckung auf.

Durch die Abdeckung im Bereich der Kreuzung der in den Führungsbereichen des Kreuzungselements geführten Spannungselemente, insbesondere im Bereich der Kreuzung des in einem ersten Führungsbereich geführten Spannungselements mit dem in einem zweiten Führungsbereich geführten Spannungselement, wird das mindestens eine sich in dem Kreuzungselement kreuzenden Spannungselement des Hilfsmittels vorteilhafterweise vor äußeren Einflüssen geschützt. Insbesondere bietet die Abdeckung einen Schutz vor unerwünschtem Druck auf den mindestens einen Kreuzungspunkt des mindestens einen Spannungselements des Hilfsmittels.

In einer bevorzugten Ausführungsform der vorliegenden Erfindung weist die Abdeckung des Kreuzungselements ebenfalls mindestens einen weiteren Führungsbereich auf, in dem ein Spannungselement des Hilfsmittels geführt werden kann, insbesondere berührungsfrei über die weiteren in dem Kreuzungselement verlaufenden Spannungselemente geführt werden kann. Gemäß dieser Ausführungsform kann vorgesehen sein, dass sich Spannungselemente, die in dem mindestens einen ersten Führungsbereich und dem mindestens einen zweiten Führungsbereich des Kreuzungselements und in dem mindestens einen weiteren Führungsbereichs der Abdeckung geführt werden, in mindestens drei unterschiedlichen Ebenen kreuzen, insbesondere berührungsfrei kreuzen.

Gemäß einer weiteren bevorzugten Ausführungsform der vorliegenden Erfindung ist die Abdeckung des Kreuzungselements abnehmbar. Erfindungsgemäß kann auch vorgesehen sein, dass die Abdeckung nicht abnehmbar ist, insbesondere dass die Abdeckung nicht zerstörungsfrei von dem Kreuzungselement entfernt werden kann.

Bevorzugt ist die Abdeckung des Kreuzungselements drehbar gelagert. Durch die Drehbarkeit der Abdeckung können an dem mindestens einen Spannungselement des Hilfsmittels anliegende Kräfte besser verteilt werden.

Gemäß der vorliegenden Erfindung kann es sich bei den in den mindestens zwei Führungsbereichen des Kreuzungselements geführten Spannungselementen, insbesondere bei dem in dem ersten Führungsbereich geführten Spannungselement und bei dem in dem zweiten Führungsbereich geführten Spannungselement, um dasselbe Spannungselement des Hilfsmittels handeln, insbesondere um ein einzelnes zusammenhängendes Spannungselement des Hilfsmittels. Erfindungsgemäß kann auch vorgesehen sein, dass es sich bei den in den mindestens zwei Führungsbereichen des Kreuzungselements geführten Spannungselementen, insbesondere bei dem in dem ersten Führungsbereich geführten Spannungselement und bei dem in dem zweiten Führungsbereich geführten Spannungselement, um unterschiedliche Spannungselemente des Hilfsmittels handelt, insbesondere um nicht miteinander verbundene Spannungselemente des Hilfsmittels.

Bevorzugt handelt es sich bei dem in den mindestens zwei Führungsbereichen des Kreuzungselements geführten Spannungselementen, insbesondere bei dem in dem ersten Führungsbereich des Kreuzungselements geführten Spannungselement und bei dem in dem zweiten Führungsbereich des Kreuzungselements geführten Spannungselement, um dasselbe Spannungselement des Hilfsmittels. Gemäß dieser Ausführungsform handelt es sich bei den in den mindestens zwei Führungsbereichen des Kreuzungselements geführten Spannungselementen, insbesondere bei dem in dem ersten Führungsbereich des Kreuzungselements geführten Spannungselement und bei dem in dem zweiten Führungsbereich des Kreuzungselements geführten Spannungselement, um ein einzelnes zusammenhängenden Spannungselement des Hilfsmittels, das sich in dem erfindungsgemäßen Kreuzungselement mindestens einmal berührungsfrei kreuzt. Entsprechend kann beispielsweise vorgesehen sein, dass ein einzelnes Spannungselement in einem Hilfsmittel so verläuft, dass sich unterschiedliche Abschnitte des Spannungselements in dem erfindungsgemäßen Kreuzungselement berührungsfrei kreuzen.

In einer weiteren Ausführungsform der vorliegenden Erfindung handelt es sich bei den in den mindestens zwei Führungsbereichen des Kreuzungselements geführten Spannungselementen, insbesondere bei dem in dem ersten Führungsbereich des Kreuzungselements geführten Spannungselement und bei dem in dem zweiten Führungsbereich des Kreuzungselements geführten Spannungselement, um unterschiedliche Spannungselemente des Hilfsmittels. Gemäß dieser Ausführungsform handelt es sich bei den in den mindestens zwei Führungsbereichen des Kreuzungselements geführten Spannungselementen, insbesondere bei dem in dem ersten Führungsbereich des Kreuzungselements geführten Spannungselement und bei dem in dem zweiten Führungsbereich des Kreuzungselements geführten Spannungselement jeweils um nicht miteinander verbundene Spannungselemente des Hilfsmittels. Erfindungsgemäß kann demnach vorgesehen sein, dass es sich in dem Spannungselement, das in dem ersten Führungsbereich geführt wird, und dem Spannungselement, das in dem zweiten Führungsbereich geführt wird, um zwei nicht miteinander verbundene Spannungselemente eines Hilfsmittels handelt, die sich in dem erfindungsgemäßen Kreuzungselement berührungsfrei kreuzen.

Gemäß einer bevorzugten Ausführungsform ist das mindestens eine Spannungselement, insbesondere das in dem ersten Führungsbereich geführte Spannungselement und/oder das in dem zweiten Führungsbereich des geführte Spannungselement, elastisch, teilelastisch oder unelastisch.

In einer bevorzugten Ausführungsform der vorliegenden Erfindung ist das mindestens eine Spannungselement, insbesondere das in dem ersten Führungsbereich des Kreuzungselements geführte Spannungselement des Hilfsmittels und/oder das in dem zweiten Führungsbereich des Kreuzungselements geführte Spannungselement des Hilfsmittels, ausgewählt aus der Gruppe bestehend aus Spann- oder Zuggurt, Spann- oder Zugseil, Spann- oder Zugband, und gliederartiges Spann- oder Zugelement.

Bevorzugt handelt es sich bei dem mindestens einen Spannungselement, insbesondere bei dem in dem ersten Führungsbereich geführten Spannungselement des Hilfsmittels und/oder bei dem in dem zweiten Führungsbereich geführten Spannungselement des Hilfsmittels, um ein Spann- oder Zuggurt. In einer weiteren Ausführungsform handelt es sich bei dem mindestens einen Spannungselement, insbesondere bei dem in dem ersten Führungsbereich geführten Spannungselement des Hilfsmittels und/oder bei dem in dem zweiten Führungsbereich geführten Spannungselement des Hilfsmittels, um ein Spann- oder Zugseil. Bevorzugt handelt es sich bei dem mindestens einen Spannungselement, insbesondere bei dem in dem ersten Führungsbereich geführten Spannungselement des Hilfsmittels und/oder bei dem in dem zweiten Führungsbereich geführten Spannungselement des Hilfsmittels, um ein Spann- oder Zugband. In einer weiteren Ausführungsform der vorliegenden Erfindung handelt es sich bei dem mindestens einen Spannungselement, insbesondere bei dem in dem ersten Führungsbereich geführten Spannungselement des Hilfsmittels und/oder bei dem in dem zweiten Führungsbereich geführten Spannungselement des Hilfsmittels, um ein gliederartiges Spann- oder Zugelement.

In einer bevorzugten Ausführungsform der vorliegenden Erfindung kann vorgesehen sein, dass das mindestens eine Spannungselement, insbesondere das in dem ersten Führungsbereich geführte Spannungselement und/oder das in dem zweiten Führungsbereich geführte Spannungselement, des Hilfsmittels vollständig oder teilweise, insbesondere abschnittsweise, ummantelt ist.

In einer besonders bevorzugten Ausführungsform der vorliegenden Erfindung handelt es sich bei dem mindestens einen Spannungselement, insbesondere bei dem in dem ersten Führungsbereich geführten Spannungselement und/oder bei dem in dem zweiten Führungsbereich geführten Spannungselement, um einen Bowdenzug.

In einer bevorzugten Ausführungsform der vorliegenden Erfindung ist das Kreuzungselement in ein orthopädisches oder Sport-Hilfsmittel integriert. Besonders bevorzugt kann das Kreuzungselement nicht zerstörungsfrei von dem orthopädischen oder Sport-Hilfsmittel getrennt werden.

Gemäß einer weiteren bevorzugten Ausführungsform der vorliegenden Erfindung kann das Kreuzungselement aus dem orthopädischen oder Sport-Hilfsmittel entnommen werden.

Gegenstand der vorliegenden Erfindung ist ferner ein orthopädisches oder Sport-Hilfsmittel umfassend mindestens ein erfindungsgemäßes Kreuzungselement.

Gemäß einer bevorzugten Ausführungsform umfasst das orthopädische oder Sport-Hilfsmittel mindestens zwei, bevorzugt mindestens drei, bevorzugt mindestens vier, Kreuzungselemente. Bevorzugt umfasst das orthopädische oder Sport-Hilfsmittel höchstens zehn, bevorzugt höchstens neun, bevorzugt höchstens acht, bevorzugt höchstens sieben, bevorzugt höchstens sechs, bevorzugt höchstens fünf, bevorzugt höchstens vier, bevorzugt höchstens drei, bevorzugt höchstens zwei, Kreuzungselemente. In einer weiteren bevorzugten Ausführungsform umfasst das orthopädische oder Sport-Hilfsmittel zwei, bevorzugt drei, bevorzugt vier, bevorzugt fünf, Kreuzungselemente.

In einer bevorzugten Ausführungsform der vorliegenden Erfindung ist das orthopädische oder Sport-Hilfsmittel ausgewählt aus der Gruppe bestehend aus Rückenorthese, Rückenbandage, Hüftorthese, Hüftbandage, Kniegelenkorthese, Kniegelenkbandage, Ellenbogenorthese, Ellenbogenbandage, Armorthese, Armbandage, Sprunggelenkorthese, Sprunggelenkbandage, Handschuhe und Schuhe. Besonders bevorzugt ist das orthopädische oder Sport-Hilfsmittel ausgewählt aus der Gruppe bestehend aus Rückenorthese, Rückenbandage, Hüftorthese, Hüftbandage, Kniegelenkorthese, Kniegelenkbandage, Ellenbogenorthese, Ellenbogenbandage, Armorthese, Armbandage, Sprunggelenkorthese und Sprunggelenkbandage.

Bevorzugt handelt es sich bei dem orthopädischen oder Sport-Hilfsmittel um eine Rückenorthese. In einer bevorzugten Ausführungsform der Erfindung handelt es sich bei dem orthopädischen oder Sport-Hilfsmittel um eine Rückenbandage. Gemäß einer besonders bevorzugten Ausführungsform handelt es sich bei dem orthopädischen oder Sport-Hilfsmittel um eine Hüftorthese. Bevorzugt handelt es sich bei dem orthopädischen oder Sport-Hilfsmittel um eine Hüftbandage. Erfindungsgemäß kann auch vorgesehen sein, dass es sich bei dem orthopädischen oder Sport-Hilfsmittel um eine Kniegelenkorthese handelt. Bevorzugt handelt es sich bei dem orthopädischen oder Sport-Hilfsmittel um eine Kniegelenkbandage. Gemäß einer bevorzugten Ausführungsform ist das orthopädische oder Sport-Hilfsmittel eine Armorthese. Bevorzugt ist das orthopädische oder Sport-Hilfsmittel eine Armbandage. In einer weiteren Ausführungsform handelt es sich bei dem orthopädischen oder Sport-Hilfsmittel um eine Sprunggelenkorthese. Gemäß einer weiteren bevorzugten Ausführungsform der Erfindung handelt es sich bei dem orthopädischen oder Sport-Hilfsmittel um eine Sprunggelenkbandage. Bevorzugt ist das orthopädische oder Sport-Hilfsmittel ein Handschuh. In einer besonders bevorzugten Ausführungsform der vorliegenden Erfindung handelt es sich bei dem orthopädischen oder Sport-Hilfsmittel um einen Schuh, insbesondere um einen Sportschuh, beispielsweise um einen Joggingschuh, einen Wanderschuh, einen Fahrradschuh, einen Skischuh oder Snowboard Boots.

Gemäß einer bevorzugten Ausführungsform der vorliegenden Erfindung ist das mindestens eine Kreuzungselement des orthopädischen oder Sport-Hilfsmittels drehbar gelagert. Besonders bevorzugt ist das mindestens eine Kreuzungselement des orthopädischen oder Sport-Hilfsmittels um eine Drehachse, insbesondere um eine zentrale Drehachse, drehbar gelagert. Durch die Drehbarkeit des Kreuzungselements und damit verbunden des ersten und zweiten Führungsbereichs, können vorteilhafterweise durch die Spannungselemente auf das Hilfsmittel wirkende Scher- und/oder Zugkräfte besser aufgenommen und verteilt werden. So kann es beispielsweise im angelegten Zustand des orthopädischen oder Sport-Hilfsmittels mit zunehmender Spannung an den Spannungselementen zu einer geringfügigen Formveränderung des orthopädischen oder Sport-Hilfsmittels kommen wodurch sich auch die Lage der sich in dem Kreuzungselement berührungsfrei kreuzenden Spannungselemente zueinander verändern kann. Eine Drehbarkeit des Kreuzungselements kann Formveränderungen des Hilfsmittels ausgleichen und die durch die Spannungselemente auf das Hilfsmittel wirkenden Kräfte gleichmäßig verteilen.

Die vorliegende Erfindung betrifft auch die Verwendung eines erfindungsgemäßen Kreuzungselements zum berührungsfreien Kreuzen mindestens eines Spannungselements eines orthopädischen oder Sport-Hilfsmittels.

Die im Zusammenhang mit dem erfindungsgemäßen Kreuzungselement beschriebenen Ausführungsformen und getroffenen Aussagen betreffen *mutatis mutandis* auch das erfindungsgemäße orthopädische oder Sport-Hilfsmittel und die erfindungsgemäße Verwendung des Kreuzungselements.

Weitere Ausführungsformen der vorliegenden Erfindung ergeben sich aus den Unteransprüchen.

Der Begriff "Kreuzungselement" bezeichnet im Zusammenhang mit der vorliegenden Erfindung eine Struktur an der oder innerhalb derer sich eine berührungsfreie Kreuzung von mindestens zwei Spannungselementen verwirklichen lässt. Dabei kann es sich erfindungsgemäß um eine ein-, zwei- , oder mehrteilig aufgebaute Struktur handeln.

Im Zusammenhang mit der vorliegenden Erfindung wird unter einem "orthopädischen oder Sport-Hilfsmittel" einerseits ein an den menschlichen oder tierischen Körper anlegbares orthopädisches Hilfsmittel zur Behandlung und Korrektur angeborener Fehlstellungen, zur Prävention und Therapie chronischer Erkrankungen sowie zur Behandlung von Verletzungen des Stütz- und Bewegungsapparates verstanden. Die Bezeichnung erfasst erfindungsgemäß zudem ein an den menschlichen oder tierischen Körper anlegbares Hilfsmittel für die Unterstützung, Stabilisierung und/oder Schonung des Stütz- und Bewegungsapparates, insbesondere zur Unterstützung, Stabilisierung und/oder Schonung des Bandapparat, der Gelenke und/oder der Muskeln, während verstärkter physischer Aktivität, insbesondere während sportlicher Betätigung. Von dem Begriff "Sport-Hilfsmittel" sind erfindungsgemäß demnach auch an den Körper anlegbare Sportartikel erfasst, beispielsweise Handschuhe und Schuhe. Bevorzugt handelt es sich bei dem "orthopädischen oder Sport-Hilfsmittel" um eine äußerlich an den menschlichen oder tierischen Körper anlegbare und mindestens eine Extremität zumindest teilweise umfassende Stützvorrichtung. Besonders bevorzugt ist das "orthopädische oder Sport-Hilfsmittel" gemäß der vorliegenden Erfindung eine Orthese oder eine Bandage.

Unter dem Begriff "Führungsbereich" wird im Zusammenhang mit der vorliegenden Erfindung ein räumlich abgrenzbarer Teil eines Kreuzungselements verstanden, der dazu geeignet ist, ein Spannungselement zu führen, das heißt, die Beweglichkeit des Spannungselements innerhalb bestimmter Grenzen zu beschränken. In besonders bevorzugter Ausführungsform ist der "Führungsbereich" des erfindungsgemäßen Kreuzungselements in Form eines offenen oder geschlossenen Führungskanals, insbesondere in Form einer Nut, bevorzugt in Form einer sich zum Kreuzungspunkt hin verjüngenden Nut, ausgebildet.

Bei einem "Spannungselement" handelt es sich im Zusammenhang mit der vorliegenden Erfindung um eine elastische, teilelastische oder unelastische Struktur, die sich zur Aufnahme Weiterleitung, und/oder Verteilung von Kräften, insbesondere von Spann- und Zugkräften, eignet. Besonders bevorzugt handelt es sich bei dem "Spannungselement" um einen Spann- oder Zuggurt, ein Spann- oder Zugseil, ein Spann- oder Zugband oder ein gliederartiges Spann- oder Zugelement. Bei dem in dem ersten Führungsbereich geführten "Spannungselement" des Hilfsmittels und dem in dem zweiten Führungsbereich geführten "Spannungselement" kann es sich erfindungsgemäß um ein einzelnes zusammenhängenden "Spannungselement" eines Hilfsmittels handeln oder um jeweils unterschiedliche, nicht miteinander verbundene "Spannungselemente" des Hilfsmittels.

Im Zusammenhang mit der vorliegenden Erfindung wird unter den Begriffen "umfassend" und "aufweisend" verstanden, dass zusätzlich zu den von diesen Begriffen explizit erfassten Elementen noch weitere, nicht explizit genannte Elemente hinzutreten können. Im Zusammenhang mit der vorliegenden Erfindung wird unter diesen Begriffen auch verstanden, dass allein die explizit genannten Elemente erfasst werden und keine weiteren Elemente vorliegen. In dieser besonderen Ausführungsform ist die Bedeutung der Begriffe "umfassend" und "aufweisend" gleichbedeutend mit dem Begriff "bestehend aus". Darüber hinaus erfassen die Begriffe "umfassend" und "aufweisend" auch Zusammensetzungen, die neben den explizit genannten Elementen auch weitere nicht genannte Elemente enthalten, die jedoch von funktionell und qualitativ untergeordneter Natur sind. In dieser Ausführungsform sind die Begriffe "umfassend" und "aufweisend" gleichbedeutend mit dem Begriff "im Wesentlichen bestehend aus".

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: a) A oder B oder C oder b) (A und B) oder c) (A und C) oder d) (B und C) oder e) (A und B und C).

Die vorliegende Erfindung wird im Folgenden anhand exemplarischer Figuren illustriert. Der Erfindungsgedanke ist nicht auf diese Ausführungsbeispiele beschränkt.

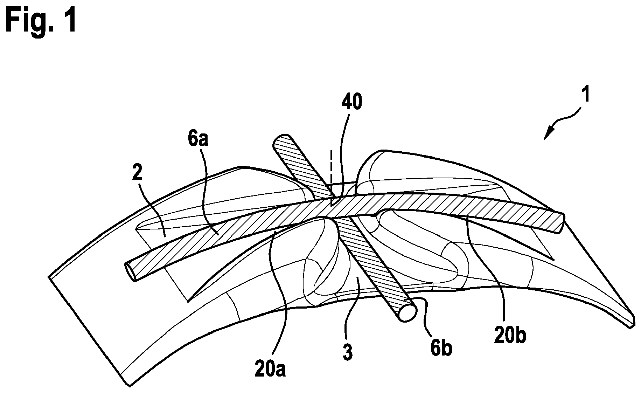

**Figur 1** zeigt eine perspektivische Darstellung eines erfindungsgemäßen Kreuzungselements (1) mit zwei Führungsbereichen (2, 3) zur Aufnahme und Führung mindestens eines sich im Kreuzungspunkt (40) berührungsfrei kreuzenden Spannungselements (6a, 6b).

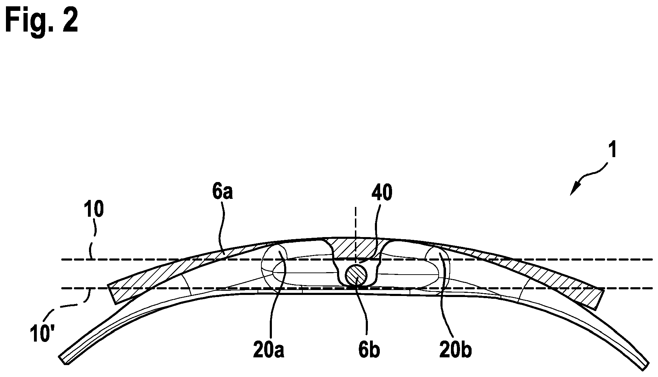

In **Figur 2** ist eine Seitenansicht eines erfindungsgemäßen Kreuzungselements (1) dargestellt. In dieser Darstellung ist ersichtlich, dass das in dem ersten Führungsbereich (2) liegende Spannungselement (6a) im Kreuzungspunkt (40) mittels zweier Rampen (20a, 20b) in einer Ebene (10) berührungsfrei über das im zweiten Führungsbereich (3) liegende und sich in einer darunterliegenden Ebene (10') befindliche Spannungselement (6b) geführt wird.

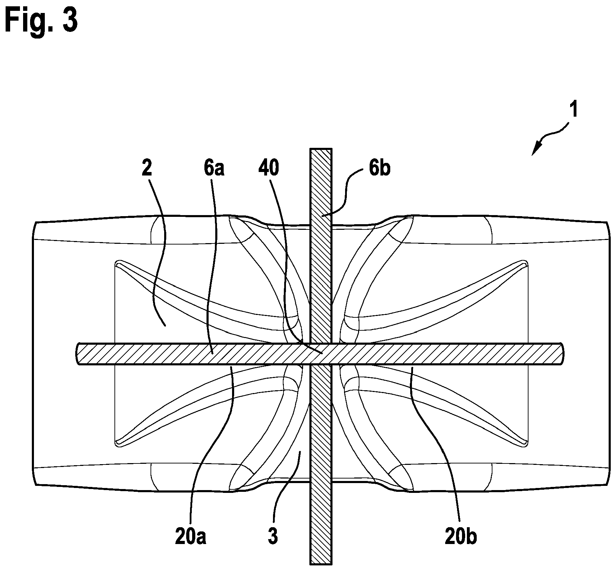

**Figur 3** zeigt eine Draufsicht auf ein erfindungsgemäßes Kreuzungselement (1). In der dargestellten Ausführungsform des Kreuzungselements (1) sind die beiden Führungsbereiche (2, 3) jeweils als sich zum Kreuzungspunkt (40) hin verjüngende Nut ausgebildet. Eine solche Ausgestaltung der Führungsbereiche (2, 3) gewährt dem in dem ersten Führungsbereich (2) liegenden Spannungselement (6a) und dem in dem zweiten Führungsbereich (3) liegenden Spannungselement (6b) in den distalen Bereichen des Kreuzungselements (1) eine gewisse Bewegungsfreiheit und schränkt die Bewegungsfreiheit zum Kreuzungspunkt (4) hin zunehmend ein, um ein Verrutschen des mindestens einen Spannungselements (6a, 6b) zu verhindern und ein berührungsfreies Kreuzen der Elemente zu gewährleisten.

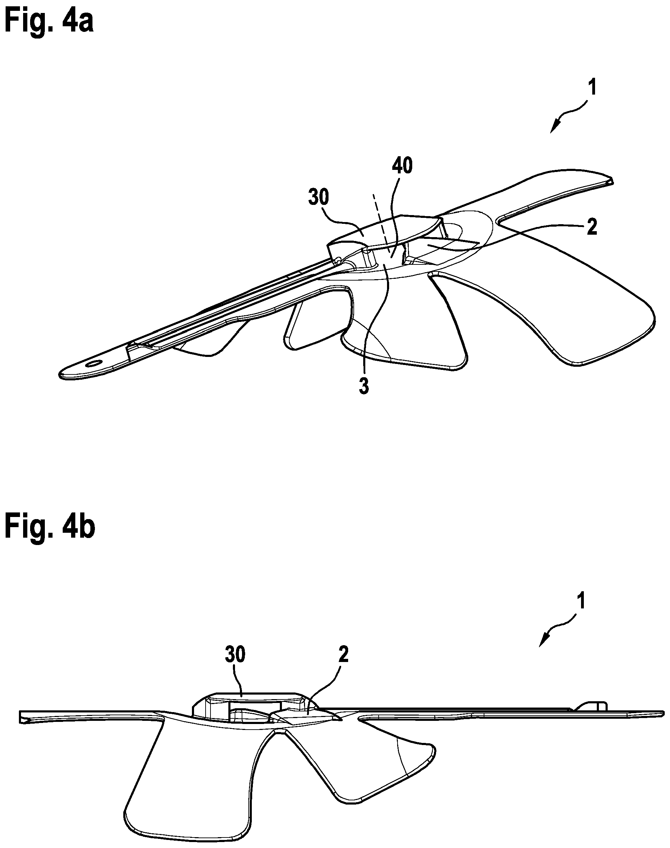

**Figur 4a** zeigt eine perspektivische Darstellung eines erfindungsgemäßen Kreuzungselements (1) für eine Rückenorthese oder Rückenbandage mit zwei Führungsbereichen (2, 3) und einer sich über den Kreuzungspunkt (40) erstreckenden Abdeckung (30). In der dargestellten Ausführungsform sind die beiden Führungsbereiche (2, 3) jeweils so breit ausgeführt, dass im Kreuzungspunkt (40) beispielsweise Spann- beziehungsweise Zuggurte berührungsfrei gekreuzt werden können. Die Abdeckung (30) dient dabei dem Schutz der Spannungselemente vor äußeren Einflüssen, insbesondere zur Verhinderung des Einwirkens von Druck auf die Spannungselemente im Kreuzungspunkt (40).

In **Figur 4b** ist eine Seitenansicht des in **Figur 4a** illustrierten Kreuzungselements (1) für eine Rückenorthese oder Rückenbandage dargestellt.

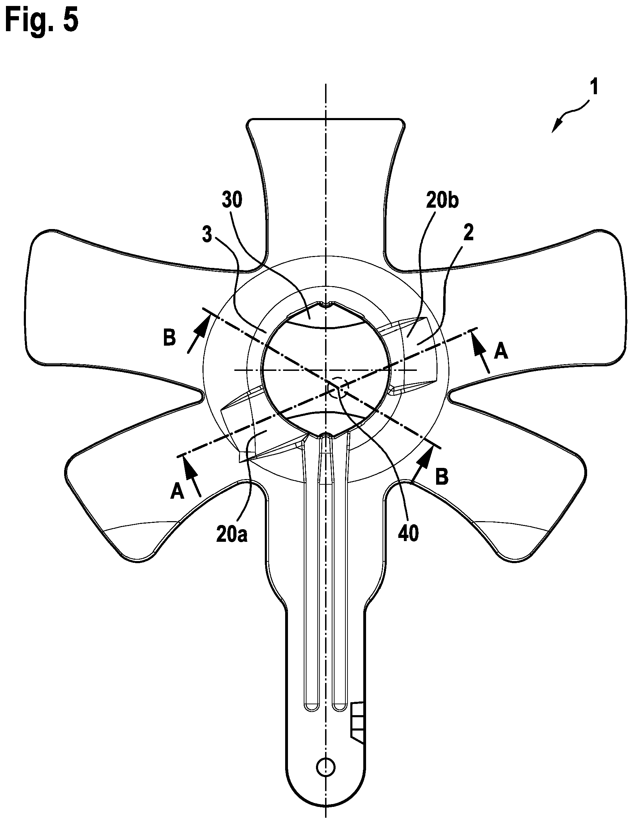

**Figur 5** zeigt eine Draufsicht auf die in den **Figuren 4a** und **4b** dargestellten Ausführungsform des erfindungsgemäßen Kreuzungselements (1). Die beiden breit ausgebildeten Führungsbereiche (2, 3) erlauben ein Kreuzen von Spann- beziehungsweise Zuggurten in einem Kreuzungspunkt (40), der von einer Abdeckung (30) geschützt ist. Dabei überquert ein in dem ersten Führungsbereich (2) geführtes Spannungselement ein in dem zweiten Führungsbereich (3) geführtes Spannungselement berührungsfrei mittels der den Kreuzungspunkt (40) flankierenden Rampen (20a, 20b).

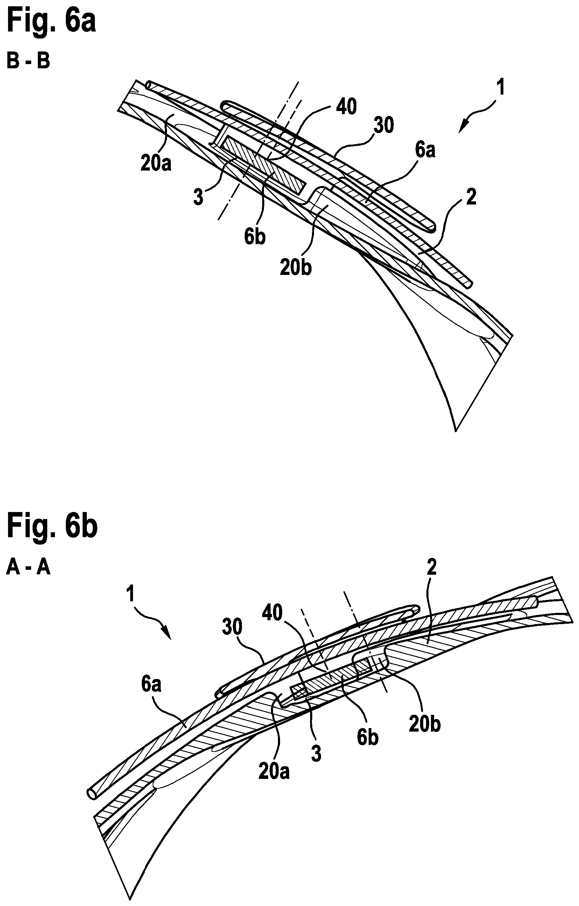

Ein Schnitt durch das in den **Figuren 4a, 4b** und **5** dargestellte Kreuzungselement (1) entlang der Schnittlinie B-B ist in **Figur 6a** illustriert. Die **Figur 6b** zeigt einen Schnitt durch das in den **Figuren 4a, 4b** und **5** dargestellte Kreuzungselement (1) entlang der Schnittlinie A-A.

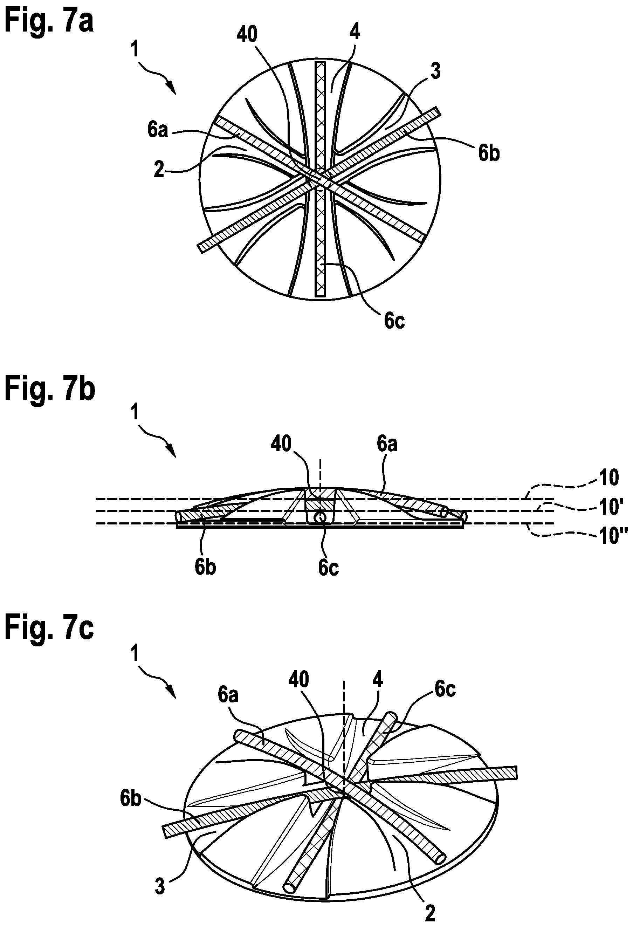

**Figur 7a** zeigt eine Draufsicht auf eine bevorzugte Ausführungsform des erfindungsgemäßen Kreuzungselements (1) mit drei Führungsbereichen (2, 3, 4) und darin geführten Spannungselementen (6a, 6b, 6c), die sich in einem zentralen Punkt (40) kreuzen. In **Figur 7b** ist eine Seitenansicht des Kreuzungselements (1) dargestellt. Die drei Spannungselemente (6a, 6b, 6c) werden im Kreuzungspunkt (40) in drei unterschiedlichen Ebenen (10, 10', 10") geführt, sodass sich die Spannungselemente (6a, 6b, 6c) berührungsfrei kreuzen. Die **Figur 7c** zeigt eine perspektivische Darstellung des Kreuzungselements (1).

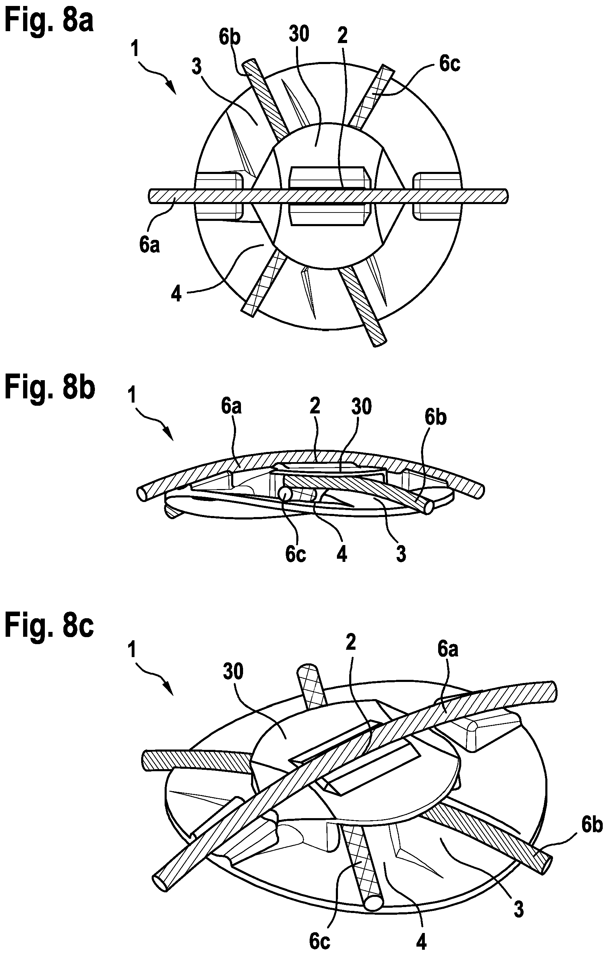

**Figur 8a** zeigt eine Draufsicht auf eine weitere Ausführungsform des erfindungsgemäßen Kreuzungselements (1). Dabei weist das Kreuzungselement (1) eine Abdeckung (30) auf, in der ein Führungsbereich (2) zur Aufnahme eines Spannungselements (6a) verläuft. Der Führungsbereich (2) der Abdeckung (30) führt das Spannungselement (6a) über zwei in den Führungsbereichen (3) und (4) verlaufende Spannungselemente (6b, 6c). Die in den Führungsbereichen (2, 3, 4) verlaufenden Spannungselemente (6a, 6b, 6c) kreuzen sich dabei berührungsfrei. In **Figur 8b** ist eine Seitenansicht der weiteren Ausführungsform des Kreuzungselements (1) dargestellt. **Figur 8c** zeigt eine perspektivische Darstellung der in den **Figuren 8a** und **8b** dargestellten Ausführungsform des Kreuzungselements (1).

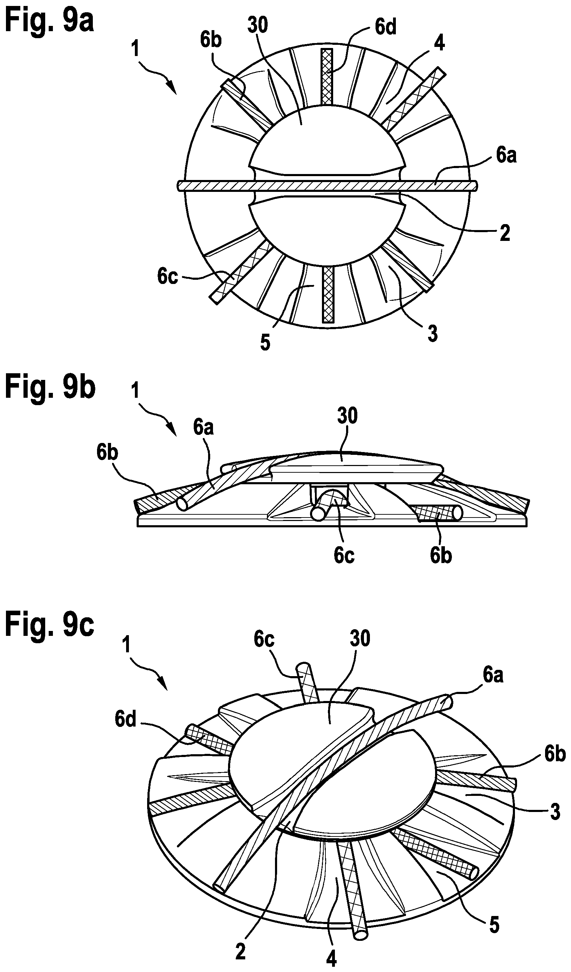

Die in den **Figur 9a, 9b** und **9c** dargestellte Ausführungsform des erfindungsgemäßen Kreuzungselements (1) erlaubt das berührungsfreie Kreuzen von vier in den Führungsbereichen (2), (3), (4) und (5) geführten Spannungselementen (6a, 6b, 6c, 6d). Der Führungsbereich (2) verläuft in der Abdeckung (30) des Kreuzungselements (1) in Form einer Nut und führt das Spannungselement (6a) über die in drei unterschiedlichen darunterliegenden Ebenen verlaufenden Spannungselemente (6b, 6c, 6d).

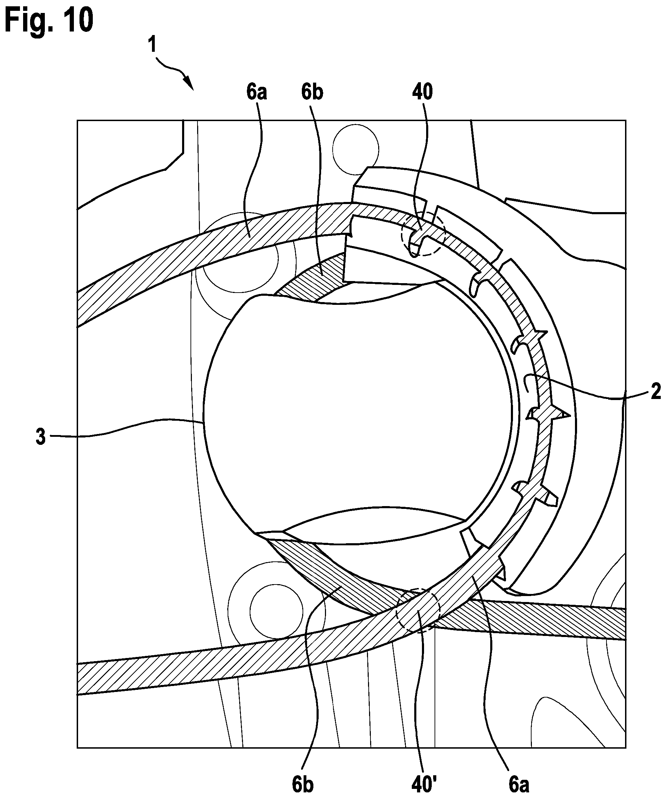

**Figur 10** zeigt eine Draufsicht auf eine weitere Ausführungsform des erfindungsgemäßen Kreuzungselements (1). Bei der dargestellten Ausführungsform handelt es sich sowohl um ein Verschlusselement als auch um ein erfindungsgemäßes Kreuzungselement (1), das bogenförmig ausgestaltete und in unterschiedlichen Ebenen verlaufende Führungsbereiche (2) und (3) aufweist, in denen Spannungselemente (6a, 6b) so geführt werden, dass diese sich in zwei unterschiedlichen Kreuzungspunkten (40, 40') des Kreuzungselements (1) berührungsfrei kreuzen. Dabei verläuft das in dem ersten Führungsbereich (2) geführte Spannungselement (6a) in den beiden Kreuzungspunkten (40, 40') über dem in dem zweiten Führungsbereich (3) geführten Spannungselement (6b).

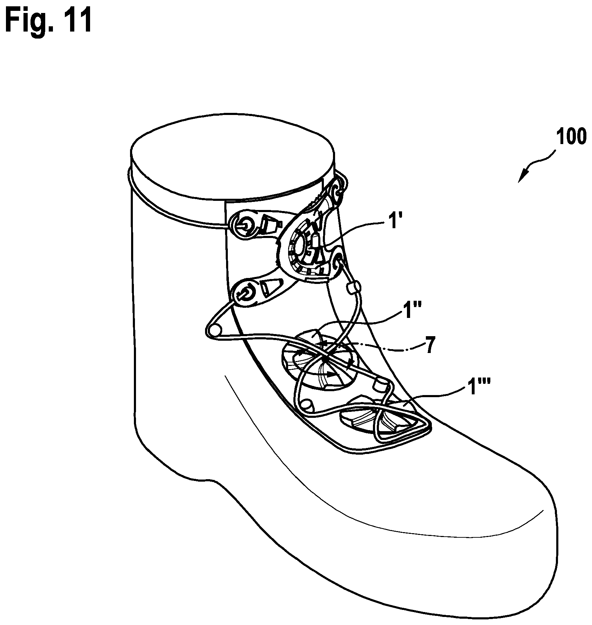

**Figur 11** zeigt ein Sport-Hilfsmittel (100) in Form eines Schuhs mit drei Kreuzungselementen (1', 1", 1‴), in denen sich Spannungselemente berührungsfrei kreuzen. Das zweite Kreuzungselement (1") ist in der dargestellten Ausführungsform um eine zentrale Drehachse (7) drehbar.

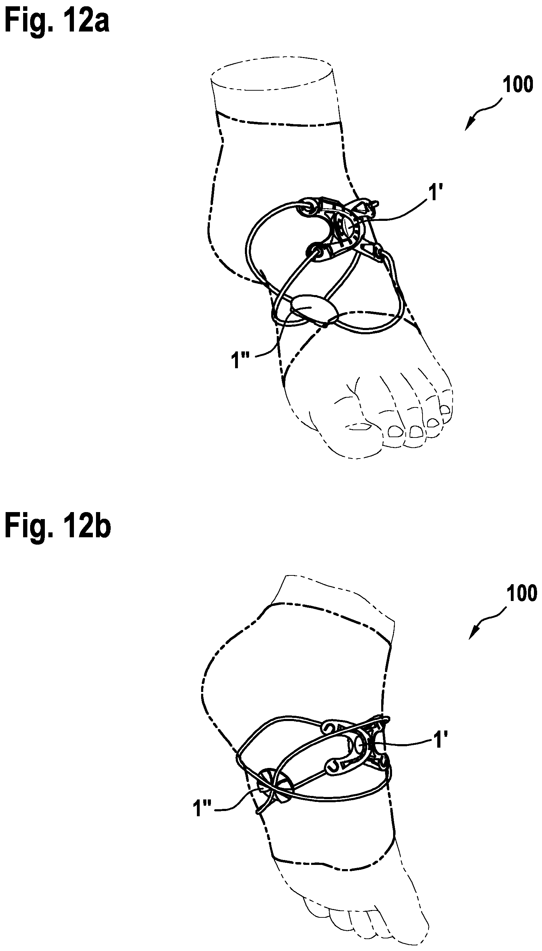

In den **Figuren 12a** und **12b** ist eine Sprunggelenkorthese/-bandage (100) mit zwei Kreuzungselementen (1', 1") aus zwei unterschiedlichen Perspektiven dargestellt. Dabei befindet sich das erste Kreuzungselement (1') im angelegten Zustand der Sprunggelenkorthese/-bandage (100) auf dem Fußrücken (Spann) im Bereich der Mittelfußknochen. Das zweite Kreuzungselement (1") befindet sich gegenüberliegend im Bereich des Mittelgewölbes oder medialen Längsgewölbes der Fußsohle.

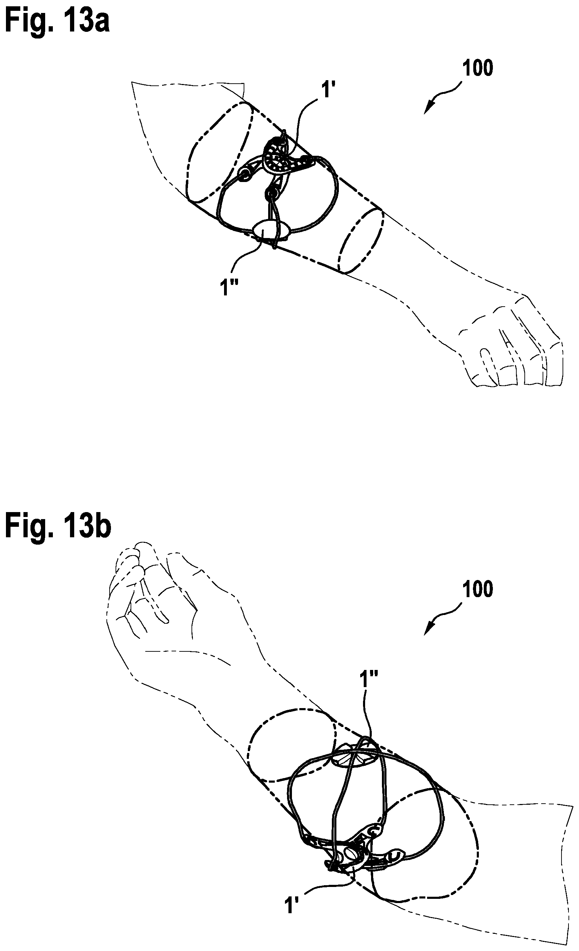

Die **Figuren 13a** und **13b** zeigen perspektivisch eine Unterarmorthese/-bandage (100) mit zwei Kreuzungselementen (1', 1"). Das erste Kreuzungselement (1') befindet sich im angelegten Zustand der Unterarmorthese/-bandage (100) auf der Außenseite des Unterarmes. Das zweite Kreuzungselement (1") ist gegenüberliegend auf der Innenseite des Unterarmes angeordnet.

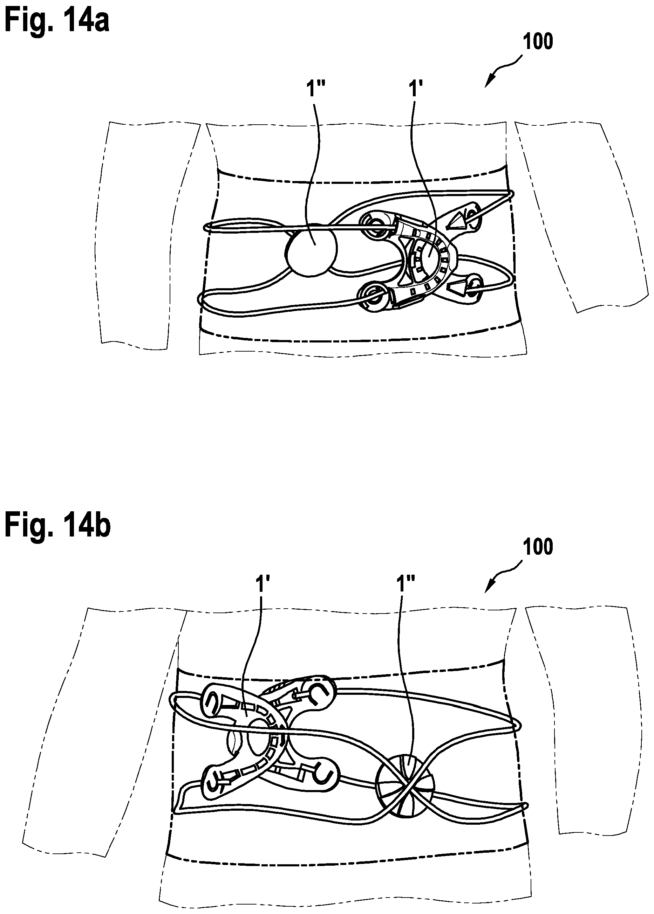

**Figuren 14a** und **14b** zeigen unterschiedliche Perspektiven auf eine erfindungsgemäße Rückenorthese/-bandage (100) mit zwei einander gegenüberliegenden Kreuzungselementen (1', 1"), in denen sich Spannungselemente berührungsfrei kreuzen. In der dargestellten Ausführungsform befindet sich das erste Kreuzungselement (1') auf etwa Höhe des Bauchnabels und das zweite Kreuzungselement (1") auf der Seite des Rückens im Bereich der Lendenwirbelsäule.

### Bezugszeichenliste

- 1: Kreuzungselement
- 1': erstes Kreuzungselement
- 1": zweites Kreuzungselement
- 1‴: drittes Kreuzungselement
- 2: erster Führungsbereich
- 3: zweiter Führungsbereich
- 4: dritter Führungsbereich
- 5: vierter Führungsbereich
- 6a: Spannungselement
- 6b: Spannungselement
- 6c: Spannungselement
- 6d: Spannungselement
- 7: Drehachse
- 10: erste Ebene
- 10': zweite Ebene
- 10": dritte Ebene
- 20a: Rampe
- 20b: Rampe
- 30: Abdeckung
- 40: Kreuzungspunkt
- 40': Kreuzungspunkt
- 100: Orthopädisches oder Sport-Hilfsmittel

## Patentansprüche

1. Kreuzungselement (1) für ein orthopädisches oder Sport-Hilfsmittel, umfassend mindestens einen ersten Führungsbereich (2) zur Aufnahme eines Spannungselements (6a) des Hilfsmittels und mindestens einen zweiten Führungsbereich (3) zur Aufnahme eines Spannungselements (6b) des Hilfsmittels, **dadurch gekennzeichnet, dass**
die mindestens zwei Führungsbereiche (2, 3) des Kreuzungselements (1) so ausgestaltet und zueinander angeordnet sind, dass sich ein in dem ersten Führungsbereich (2) geführtes Spannungselement (6a) und ein in dem zweiten Führungsbereich (3) geführtes Spannungselement (6b) in mindestens einem Punkt (40) berührungsfrei kreuzen.

2. Kreuzungselement (1) nach Anspruch 1, **dadurch gekennzeichnet, dass** es sich bei dem ersten (2) und/oder dem zweiten Führungsbereich (3) des Kreuzungselements um einen Führungskanal handelt, wobei der Führungskanal bevorzugt in Form einer Nut ausgebildet ist.

3. Kreuzungselement (1) nach einem der vorhergehenden Ansprüche, **dadurch gekennzeichnet, dass** der erste und/oder zweite Führungsbereich (2, 3) so ausgestaltet ist, dass ein in dem ersten und/oder zweiten Führungsbereich (2, 3) geführtes Spannungselement (6a, 6b) zumindest teilweise umgriffen wird.

4. Kreuzungselement (1) nach einem der vorhergehenden Ansprüche, **dadurch gekennzeichnet, dass** der erste und der zweite Führungsbereich (2, 3) zumindest im Bereich des mindestens einen Kreuzungspunktes (40) des in dem ersten Führungsbereich (2) geführten Spannungselements (6a) mit dem in dem zweiten Führungsbereich (3) geführten Spannungselement (6b) in unterschiedlichen Ebenen (10, 10') liegen.

5. Kreuzungselement (1) nach einem der vorhergehenden Ansprüche, **dadurch gekennzeichnet, dass** der erste Führungsbereich (2) zwei Rampen (20a, 20b) umfasst, wobei der zweite Führungsbereich (3) zwischen den zwei Rampen (20a, 20b) des ersten Führungsbereichs (2) verläuft, sodass ein im ersten Führungsbereich (2) verlaufendes Spannungselement (6a) mittels der zwei Rampen (20a, 20b) über ein im zweiten Führungsbereich (3) verlaufendes Spannungselement (6b) geführt wird.

6. Kreuzungselement (1) nach einem der vorhergehenden Ansprüche, **dadurch gekennzeichnet, dass** das Kreuzungselement (1) zumindest über dem mindestens einen Kreuzungspunkt (40) des in dem ersten Führungsbereich (2) geführten Spannungselements (6a) mit dem in dem zweiten Führungsbereich (3) geführten Spannungselement (6b) mindestens eine Abdeckung (30) aufweist.

7. Kreuzungselement (1) nach Anspruch 6, **dadurch gekennzeichnet, dass** die Abdeckung (30) des Kreuzungselements (1) mindestens einen weiteren Führungsbereich aufweist, in dem ein Spannungselement des Hilfsmittels geführt werden kann.

8. Kreuzungselement (1) nach Anspruch 6 oder 7, **dadurch gekennzeichnet, dass** die Abdeckung (30) abnehmbar ist.

9. Kreuzungselement (1) nach einem der Ansprüche 6 bis 8, **dadurch gekennzeichnet, dass** die Abdeckung (30) drehbar gelagert ist.

10. Kreuzungselement (1) nach einem der vorhergehenden Ansprüche, **dadurch gekennzeichnet, dass** es sich bei dem in dem ersten Führungsbereich (2) geführten Spannungselement (6a) und bei dem in dem zweiten Führungsbereich (3) geführten Spannungselement (6b) um dasselbe Spannungselement des Hilfsmittels handelt, insbesondere um ein einzelnes zusammenhängendes Spannungselement des Hilfsmittels.

11. Kreuzungselement (1) nach einem der Ansprüche 1 bis 9, **dadurch gekennzeichnet, dass** es sich bei dem in dem ersten Führungsbereich (2) geführten Spannungselement (6a) und bei dem in dem zweiten Führungsbereich (3) geführten Spannungselement (6b) um unterschiedliche Spannungselemente des Hilfsmittels handelt, insbesondere um nicht miteinander verbundene separate Spannungselemente des Hilfsmittels.

12. Orthopädisches oder Sport-Hilfsmittel (100) umfassend mindestens ein Kreuzungselement (1) nach einem der Ansprüche 1 bis 11.

13. Orthopädisches oder Sport-Hilfsmittel (100) nach Anspruch 12, **dadurch gekennzeichnet, dass** das Hilfsmittel (100) mindestens ein Spannungselement aufweist, wobei das Spannungselement ausgewählt ist aus der Gruppe bestehend aus Spann- oder Zuggurt, Spann- oder Zugseil, Spann- oder Zugband und gliederartigem Spann- oder Zugelement.

14. Orthopädisches oder Sport-Hilfsmittel (100) nach Anspruch 12 oder 13, ausgewählt aus der Gruppe bestehend aus Rückenorthese, Rückenbandage Hüftorthese, Hüftbandage, Kniegelenkorthese, Kniegelenkbandage, Ellenbogenorthese, Ellenbogenbandage, Armorthese, Armbandage, Sprunggelenkorthese, Sprunggelenkbandage, Handschuhe und Schuhe.

15. Orthopädisches oder Sport-Hilfsmittel (100) nach einem der Ansprüche 12 bis 14, **dadurch gekennzeichnet, dass** das mindestens eine Kreuzungselement (1) drehbar gelagert auf dem Hilfsmittel angeordnet ist.

## Claims

1. Crossing element (1) for an orthopedic or sports aid device, comprising at least one first guide section (2) for receiving a tensioning element (6a) of the aid device and at least one second guide section (3) for receiving a tensioning element (6b) of the aid device, **characterized in that**
the at least two guide sections (2, 3) of the crossing element (1) are designed and arranged relative to one another such that a tensioning element (6a) guided in the first guide section (2) and a tensioning element (6b) guided in the second guide section (3) cross each other without contact at at least one point (40).

2. Crossing element (1) according to claim 1, **characterized in that** the first (2) and/or the second guide section (3) of the crossing element is a guide channel, wherein the guide channel is preferably designed in the form of a groove.

3. Crossing element (1) according to one of the preceding claims, **characterized in that** the first and/or second guide section (2, 3) is designed such that a tensioning element (6a, 6b) guided in the first and/or second guide section (2, 3) is at least partially encompassed.

4. Crossing element (1) according to one of the preceding claims, **characterized in that** the first and second guide sections (2, 3) are located in different planes (10, 10') at least in the region of the at least one crossing point (40) of the tensioning element (6a) guided in the first guide section (2) with the tensioning element (6b) guided in the second guide section (3).

5. Crossing element (1) according to one of the preceding claims, **characterized in that** the first guide section (2) comprises two ramps (20a, 20b), wherein the second guide section (3) runs between the two ramps (20a, 20b) of the first guide section (2) such that a tensioning element (6a) running in the first guide section (2) is guided by means of the two ramps (20a, 20b) above a tensioning element (6b) running in the second guide section (3).

6. Crossing element (1) according to one of the preceding claims, **characterized in that** the crossing element (1) comprises at least one cover (30) above the at least one crossing point (40) of the tensioning element (6a) guided in the first guide section (2) with the tensioning element (6b) guided in the second guide section (3).

7. Crossing element (1) according to claim 6, **characterized in that** the cover (30) of the crossing element (1) comprises at least one further guide section in which a tensioning element of the aid device can be guided.

8. Crossing element (1) according to claim 6 or 7, **characterized in that** the cover (30) is removable.

9. Crossing element (1) according to one of claims 6 to 8, **characterized in that** the cover (30) is rotatably mounted.

10. Crossing element (1) according to one of the preceding claims, **characterized in that** the tensioning element (6a) guided in the first guide section (2) and the tensioning element (6b) guided in the second guide section (3) are the same tensioning element of the aid device, in particular a single connected tensioning element of the aid device.

11. Crossing element (1) according to one of claims 1 to 9, **characterized in that** the tensioning element (6a) guided in the first guide section (2) and the tensioning element (6b) guided in the second guide section (3) are different tensioning elements of the aid device, in particular separate tensioning elements of the aid device which are not connected to one another.

12. Orthopedic or sports aid device (100) comprising at least one crossing element (1) according to one of claims 1 to 11.

13. Orthopedic or sports aid device (100) according to claim 12, **characterized in that** the aid device (100) comprises at least one tensioning element, wherein the tensioning element is selected from the group consisting of tensioning or pulling strap, tensioning or pulling rope, tensioning or pulling band, and limb-like tensioning or pulling element.

14. Orthopedic or sports aid device (100) according to claim 12 or 13, selected from the group consisting of back orthosis, back bandage, hip orthosis, hip bandage, knee joint orthosis, knee joint bandage, elbow orthosis, elbow bandage, arm orthosis, arm bandage, ankle joint orthosis, ankle joint bandage, gloves, and shoes.

15. Orthopedic or sports aid device (100) according to one of claims 12 to 14, **characterized in that** the at least one crossing element (1) is arranged rotatably mounted on the aid device.

## Revendications

1. Élément de croisement (1) pour un dispositif orthopédique ou sportif, comprenant au moins une première zone de guidage (2) destinée à recevoir un élément de tension (6a) du dispositif et au moins une deuxième zone de guidage (3) destinée à recevoir un élément de tension (6b) du dispositif, **caractérisé en ce que**
les au moins deux zones de guidage (2, 3) de l'élément de croisement (1) sont conçues et disposées les unes par rapport aux autres de telle sorte qu'un élément de tension (6a) guidé dans la première zone de guidage (2) et un élément de tension (6b) guidé dans la deuxième zone de guidage (3) se croisent sans contact en au moins un point (40).

2. Élément de croisement (1) selon la revendication 1, **caractérisé en ce que** la première (2) et/ou la deuxième zone de guidage (3) de l'élément de croisement est un canal de guidage, le canal de guidage étant de préférence réalisé sous la forme d'une rainure.

3. Élément de croisement (1) selon l'une des revendications précédentes, **caractérisé en ce que** la première et/ou la deuxième zone de guidage (2, 3) est conçue de telle sorte qu'un élément de tension (6a, 6b) guidé dans la première et/ou la deuxième zone de guidage (2, 3) soit au moins partiellement entouré.

4. Élément de croisement (1) selon l'une des revendications précédentes, **caractérisé en ce que** la première et la deuxième zones de guidage (2, 3) se situent dans des plans différents (10, 10') au moins au niveau du ou des points de croisement (40) de l'élément de tension (6a) guidé dans la première zone de guidage (2) avec l'élément de tension (6b) guidé dans la deuxième zone de guidage (3).

5. Élément de croisement (1) selon l'une des revendications précédentes, **caractérisé en ce que** la première zone de guidage (2) comprend deux rampes (20a, 20b), la deuxième zone de guidage (3) s'étendant entre les deux rampes (20a, 20b) de la première zone de guidage (2), de telle sorte qu'un élément de tension (6a) s'étendant dans la première zone de guidage (2) est guidé au moyen des deux rampes (20a, 20b) sur un élément de tension (6b) s'étendant dans la deuxième zone de guidage (3).

6. Élément de croisement (1) selon l'une des revendications précédentes, **caractérisé en ce que** l'élément de croisement (1) comporte au moins un cache (30) au-dessus d'au moins un point de croisement (40) de l'élément de tension (6a) guidé dans la première zone de guidage (2) avec l'élément de tension (6b) guidé dans la deuxième zone de guidage (3).

7. Élément de croisement (1) selon la revendication 6, **caractérisé en ce que** le cache (30) de l'élément de croisement (1) comporte au moins une autre zone de guidage dans laquelle un élément de tension du dispositif peut être guidé.

8. Élément de croisement (1) selon la revendication 6 ou 7, **caractérisé en ce que** le cache (30) est amovible.

9. Élément de croisement (1) selon l'une des revendications 6 à 8, **caractérisé en ce que** le cache (30) est monté de manière pivotante.

10. Élément de croisement (1) selon l'une des revendications précédentes, **caractérisé en ce que** l'élément de tension (6a) guidé dans la première zone de guidage (2) et l'élément de tension (6b) guidé dans la deuxième zone de guidage (3) sont le même élément de tension du dispositif, en particulier un seul élément de tension continu du dispositif.

11. Élément de croisement (1) selon l'une des revendications 1 à 9, **caractérisé en ce que** l'élément de tension (6a) guidé dans la première zone de guidage (2) et l'élément de tension (6b) guidé dans la deuxième zone de guidage (3) sont des éléments de tension différents du dispositif, en particulier des éléments de tension séparés du dispositif qui ne sont pas reliés entre eux.

12. Dispositif orthopédique ou sportif (100) comprenant au moins un élément de croisement (1) selon l'une des revendications 1 à 11.

13. Dispositif orthopédique ou sportif (100) selon la revendication 12, **caractérisé en ce que** le dispositif (100) comporte au moins un élément de tension, l'élément de tension étant choisi parmi le groupe constitué d'une sangle de tension ou de traction, d'un câble de tension ou de traction, d'une bande de tension ou de traction et d'un élément de tension ou de traction en forme de maillon.

14. Dispositif orthopédique ou sportif (100) selon la revendication 12 ou 13, choisi parmi le groupe constitué d'une orthèse dorsale, d'un bandage dorsal, d'une orthèse de hanche, d'un bandage de hanche, d'une orthèse de genou, d'un bandage de genou, d'une orthèse de coude, d'un bandage de coude, d'une orthèse de bras, d'un bandage de bras, d'une orthèse de cheville, d'un bandage de cheville, gants et chaussures.

15. Dispositif orthopédique ou sportif (100) selon l'une des revendications 12 à 14, **caractérisé en ce que** ledit au moins un élément de croisement (1) est monté de manière pivotante sur le dispositif.
